# FASCICULE DE BREVET EUROPEEN

(11) **EP 3 019 118 B1**
(45) Date de publication et mention de la délivrance du brevet: **01.03.2017**
(21) Numéro de dépôt: 14736898.9
(22) Date de dépôt: 12.06.2014
(51) Int. Cl.: A61D 7/00, A61D 7/04, A61M 5/31, A61M 5/20

(54) **DISPOSITIF D'INJECTION DE FLUIDE AUX ANIMAUX, NOTAMMENT PAR VOIE INTRANASALE**
VORRICHTUNG ZUM EINSPRITZEN EINES FLUIDS BEI TIEREN, INSBESONDERE DURCH INTRANASALE VERABREICHUNG
DEVICE FOR INJECTING FLUID IN ANIMALS, IN PARTICULAR BY INTRANASAL ADMINISTRATION

(30) Priorité: 12.07.2013 FR 1356847
(43) Date de publication de la demande: 18.05.2016
(73) Titulaire: Genia, 44680 Saint Hilaire De Chaleons (FR)
(72) Inventeur: COURTOIS, Christophe, 85260 L'Herbergement (FR)
(74) Mandataire: Godineau, Valérie
(86) Numéro de dépôt international: PCT/FR2014/051437
(87) Numéro de publication internationale: WO 2015/004351

(56) Documents cités:
- EP-A2- 2 033 595
- WO-A1-2012/127366
- GB-A- 1 402 428
- US-A1- 2004 116 870
- US-B1- 7 611 495

## Description

La présente invention concerne un dispositif d'injection de fluide aux animaux, notamment par voie intranasale.

Elle concerne plus particulièrement un dispositif d'injection comprenant un corps de seringue tubulaire dont l'extrémité distale est munie d'une sortie obturable d'évacuation de fluide, un piston équipé d'un poussoir et monté à coulissement axial dans ledit corps de seringue, ledit piston creux se prolongeant au-delà du poussoir par une zone tubulaire obturable raccordable à un réservoir d'alimentation en fluide à injecter, l'organe d'obturation de la zone tubulaire et l'organe d'obturation de la sortie d'évacuation de fluide, équipés chacun de moyens de rappel en position fermée étant respectivement configurés pour, à l'état partiellement introduit du piston dans le corps de seringue, passer de la position fermée à la position ouverte, l'un, sous l'effet du déplacement du piston dans le sens d'une sortie du corps de seringue, l'autre, sous l'effet du déplacement du piston dans le sens d'une entrée dans le corps de seringue.

Des dispositifs d'injection sont connus comme l'illustrent par exemple les documents GB-1.402.428, sur lequel se base la forme en deux parties de la revendication 1, EP-2.033.595, US-2004/116870, WO 2012/127366 et US-7.611.495.

Jusqu'à présent, classiquement, une seringue comporte, au niveau du corps de seringue, deux ailettes radiales externes disposées sur le corps de seringue, de manière diamétralement opposée, le dessous de chaque ailette, correspondant à la face de l'ailette tournée vers le fond du corps de seringue, formant une surface d'appui de la zone palmaire de l'index et du majeur de la main d'un opérateur, tandis que le pouce est utilisé pour actionner par poussée le piston.

Dans le cas d'une injection de fluide aux animaux par voie intranasale à l'aide d'un tel dispositif d'injection, on constate des risques de blessure importants, liés au fait que la tête de l'animal bouge constamment, de sorte que la main doit suivre à distance les mouvements de la tête.

Un but de la présente invention est donc de proposer un dispositif d'injection dont la conception permet, lors d'une injection par voie intranasale, de mieux suivre le mouvement de la tête des animaux et de limiter ainsi le risque de blessure de l'animal.

À cet effet, l'invention a pour objet un dispositif d'injection de fluide aux animaux, notamment par voie intranasale comprenant un corps de seringue tubulaire dont l'extrémité distale est munie d'une sortie obturable d'évacuation de fluide, un piston creux équipé d'un poussoir et monté à coulissement axial dans ledit corps de seringue, ledit piston creux se prolongeant au-delà du poussoir par une zone tubulaire obturable raccordable à un réservoir d'alimentation en fluide à injecter, l'organe d'obturation de la zone tubulaire et l'organe d'obturation de la sortie d'évacuation de fluide, équipés chacun de moyens de rappel en position fermée, étant respectivement configurés pour, à l'état partiellement introduit du piston dans le corps de seringue, passer de la position fermée à la position ouverte, l'un sous l'effet du déplacement du piston dans le sens d'une sortie du corps de seringue, l'autre sous l'effet du déplacement du piston dans le sens d'une entrée dans le corps de seringue, caractérisé en ce que le piston est équipé de moyens de rappel en position partiellement sortie du corps de seringue et en ce que le corps de seringue est bordé longitudinalement d'une oreille formée d'au moins deux ailes ou nervures radiales externes audit corps disposées de manière écartée l'une de l'autre le long d'un axe sensiblement parallèle à l'axe longitudinal du corps de seringue, l'intervalle entre lesdites ailes ou nervures délimitant une zone d'appui d'au moins un doigt apte à s'enrouler au moins partiellement autour dudit corps de seringue.

Grâce à la conception de l'oreille formée de deux ailes disposées le long d'un même axe, le corps de seringue peut être tenu à pleine main. En effet, les doigts de la main de l'opérateur, autres que le pouce, peuvent s'enrouler autour du corps de seringue, de sorte que la main peut, au cours de l'injection, venir, par sa tranche, en contact d'appui avec la partie de la tête de l'animal jouxtant les naseaux. Cet appui permet de mieux suivre les mouvements de la tête de l'animal et de limiter les risques de blessure.

Grâce au fait que le piston est équipé de moyens de rappel en position partiellement sortie du corps de seringue, le réarmement s'opère automatiquement, et le dispositif peut être utilisé pour la réalisation d'injections, par exemple de vaccinations, en série.

De préférence, la zone d'appui d'au moins un doigt, encadrée par les ailes, présente une surface d'appui sensiblement parallèle à l'axe longitudinal du corps de seringue.

De préférence, l'oreille est disposée sur une pièce rapportée sur le corps de seringue et fixée au corps de seringue, de préférence par vissage.

Il en résulte un nettoyage aisé de l'ensemble, et un diamètre hors-tout du dispositif d'injection augmenté, améliorant la prise en main du dispositif.

De préférence, la zone tubulaire et le piston forment entre eux un angle obtus.

Cet angle permet un dégagement de la zone tubulaire, autorisant le positionnement sans gêne de la main sur ledit corps de seringue.

De préférence, en position d'injection, ledit corps de seringue est disposé sensiblement horizontalement avec l'oreille surmontant ledit corps et l'angle obtus formé entre la zone tubulaire et le piston ouvert vers le ciel.

Cette configuration permet d'éviter toute aspiration d'air à l'intérieur du réservoir, au cours de l'étape de réarmement.

De préférence, la zone tubulaire se termine, côté raccordement à un réservoir, en formant un tube plongeur à extrémité libre chanfreinée ou en forme d'olive sur lequel le réservoir à raccorder est apte à se fixer.

Ce réservoir peut être formé par un simple flacon, fermé par un bouchon transperçable par le tube plongeur qui remplit la fonction d'organe de perforation.

De préférence, la zone tubulaire est munie, côté raccordement à un réservoir, au voisinage de son extrémité libre, d'une douille dite porte-réservoir à l'intérieur de laquelle le réservoir à raccorder est apte à s'insérer.

Il en résulte une meilleure stabilité et une protection du réservoir.

De préférence, la zone tubulaire est munie, entre son extrémité libre disposée côté raccordement à un réservoir et son organe d'obturation, d'au moins un évent.

Cet évent permet une mise à l'air libre du contenu du réservoir, pour permettre sa vidange.

De préférence, l'organe d'obturation de la zone tubulaire et l'organe d'obturation de la sortie d'évacuation de fluide comprennent chacun une bille, et les cavités formées, l'une, par la sortie d'évacuation de fluide, l'autre, par la zone tubulaire, comportent chacune un siège de réception dudit organe d'obturation associé.

De préférence, le poussoir comprend, sur une partie de son pourtour, un bras radial externe apte à former une zone d'appui du pouce d'un opérateur, ledit bras et l'oreille du corps de seringue étant disposés en regard l'un de l'autre, de manière décalée axialement suivant l'axe longitudinal du corps de seringue.

Ainsi, le bras radial s'étend en direction du sol, en position d'injection avec l'oreille surmontant le corps de seringue positionné horizontalement, comme décrit ci-dessus.

L'invention sera bien comprise à la lecture de la description suivante d'exemples de réalisation, en référence aux dessins annexés dans lesquels :

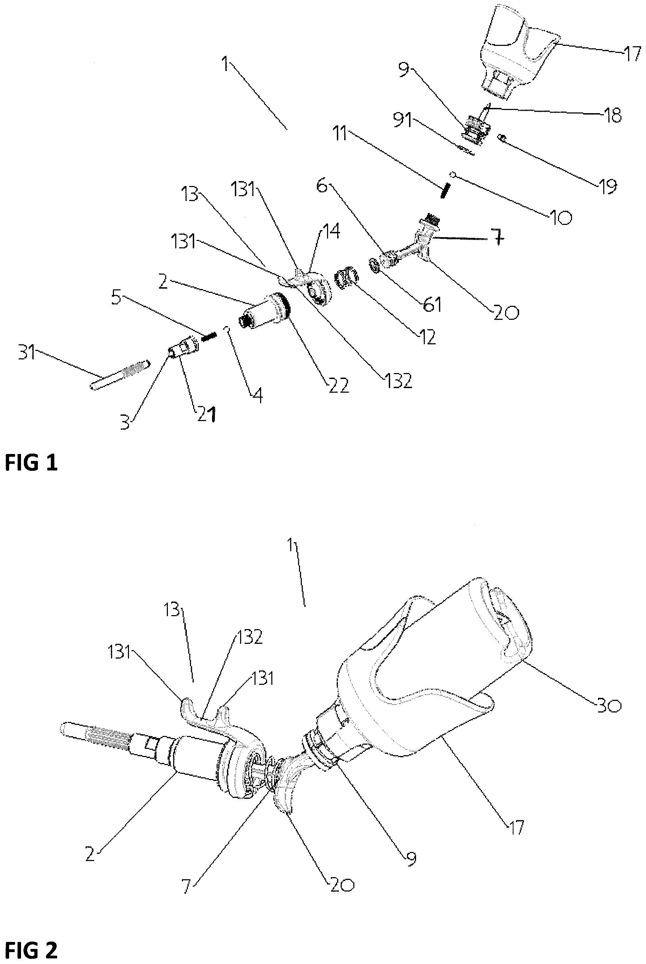

- la figure 1 représente une vue en position éclatée des éléments d'un dispositif d'injection conforme à l'invention ;
- la figure 2 représente une vue à l'état assemblé d'un dispositif d'injection conforme à l'invention ;
- la figure 3 représente une vue en perspective d'un dispositif d'injection à l'état saisi par la main d'un opérateur, en vue d'une injection par voie intranasale ;
- la figure 4 représente une vue en coupe et deux vues de détail d'un dispositif d'injection en position fermée des organes d'obturation de la zone tubulaire et de la sortie d'évacuation de fluide du corps de seringue ;
- la figure 5 représente une vue en coupe et deux vues de détail d'un dispositif d'injection en position fermée de l'organe d'obturation de la zone tubulaire et ouverte de l'organe d'obturation de la sortie d'évacuation de fluide du corps de seringue, lors d'un déplacement du piston dans le sens d'une rentrée dudit piston à l'intérieur du corps de seringue ;
- la figure 6 représente une vue en coupe et deux vues de détail d'un dispositif d'injection en position ouverte de l'organe d'obturation de la zone tubulaire et fermée de l'organe d'obturation de la sortie d'évacuation de fluide du corps de seringue, lors d'un déplacement du piston dans le sens d'une sortie dudit piston du corps de seringue.

Comme mentionné ci-dessus, le dispositif 1 d'injection objet de l'invention est destiné à tous les animaux auxquels une injection par voie intranasale peut être administrée.

Le composant à injecter est stocké dans un réservoir 30, qui peut se présenter sous forme d'un flacon fermé par un bouchon éventuellement perforable, comme cela sera décrit en détail ci-après.

Cette composition peut se présenter sous forme d'une composition prête à l'emploi ou d'une composition à reconstituer. Indépendamment de sa forme initiale, la composition à injecter se présente, au moment de l'injection, sous forme d'un fluide.

Pour permettre l'injection de ce fluide, le dispositif 1 d'injection comprend un corps 2 de seringue tubulaire, généralement cylindrique. Ce corps 2 de seringue est fermé par un fond qui forme l'extrémité 21 distale du corps. Ce fond est muni d'une sortie 3 obturable d'évacuation de fluide. L'extrémité 22 opposée proximale dudit corps de seringue est ouverte.

Dans les exemples représentés, le corps 2 de seringue est formé de plusieurs parties assemblées entre elles par vissage. Le corps 2 de seringue peut également être réalisé d'une seule pièce, sans sortir du cadre de l'invention.

La cavité formée par la sortie 3 d'évacuation de fluide comporte un siège 15 de réception de l'organe 4 d'obturation de ladite sortie 3. Cet organe 4 d'obturation est équipé de moyens 5 de rappel en position fermée de ladite sortie 3 d'évacuation de fluide.

Cet organe 4 d'obturation de la sortie 3 d'évacuation de fluide comprend une bille, et les moyens de rappel sont formés par un ressort hélicoïdal. Ces moyens de rappel, interposés ici entre l'organe d'obturation et le débouché à l'air libre de la sortie 3 d'évacuation de fluide, tendent à rappeler la bille contre son siège. Cette bille est écartée de son siège sous l'action du déplacement du piston qui sera décrit ci-après, dans le sens d'une entrée du piston dans le corps de seringue, en vue d'une injection d'une dose de fluide contenue dans le réservoir.

On note que dans l'exemple représenté, une canule 31 est raccordée à la sortie 3 d'évacuation de fluide du corps de seringue.

Comme mentionné ci-dessus, le dispositif 1 d'injection comprend encore un piston 6 formé d'une tige équipée, à l'une de ses extrémités, de la tête de piston et à son extrémité opposée, d'un poussoir 7.

Le piston 6 est introduit par sa tête de piston à l'intérieur du corps 2 de seringue, côté extrémité 22 proximale dudit corps 2 de seringue, et est monté mobile à coulissement axial dans ledit corps 2 de seringue sous l'action d'une poussée exercée par le pouce de l'opérateur sur le poussoir 7.

Ce piston 6 est un piston creux. La tige, la tête et le poussoir sont donc creux, pour délimiter une cavité longitudinale traversante dudit piston. Un joint 61 d'étanchéité torique peut être disposé autour de la tête du piston pour assurer une étanchéité entre corps de seringue et tête de piston.

Ledit piston creux se prolonge, côté opposé à la tige du piston, au-delà du poussoir 7 par une zone 9 tubulaire obturable munie d'un joint d'étanchéité 91 et raccordable à un réservoir 30 d'alimentation en fluide à injecter.

Cette zone 9 tubulaire comporte donc une cavité 100 axiale disposée dans le prolongement de la cavité 8 longitudinale du piston et en communication fluidique avec ladite cavité 8 longitudinale pour permettre le passage du fluide du réservoir de la cavité ménagée dans la zone tubulaire à la cavité longitudinale du piston, avant de parvenir dans la chambre du corps de seringue formée entre la tête de piston et le fond du corps de seringue.

Cette zone 9 tubulaire est une zone obturable, fermée par un organe 10 d'obturation équipé de moyens 11 de rappel en position fermée. Cet organe 10 d'obturation est, comme l'organe 4 d'obturation décrit ci-dessus, formé d'une bille plaquée par les moyens 11 de rappel formés par un ressort hélicoïdal en appui sur un siège 16 disposé dans la cavité 100 axiale de la zone 9 tubulaire.

La zone 9 tubulaire comporte, depuis son extrémité libre, côté raccordement au réservoir en direction de son extrémité opposée, côté raccordement au piston, le siège de l'organe d'obturation, puis l'organe d'obturation, puis les moyens de rappel.

L'organe 10 d'obturation de la zone 9 tubulaire est donc configuré pour, à l'état partiellement introduit du piston 6 dans le corps 2 de seringue, passer de la position fermée à la position ouverte, sous l'effet du déplacement du piston 6 dans le sens d'une sortie du corps 2 de seringue.

Les organes 4 et 10 d'obturation fonctionnent donc en opposition sous l'action du déplacement du piston, c'est-à-dire qu'au cours du déplacement du piston, quand l'un des organes d'obturation est ouvert, l'autre est fermé, et inversement.

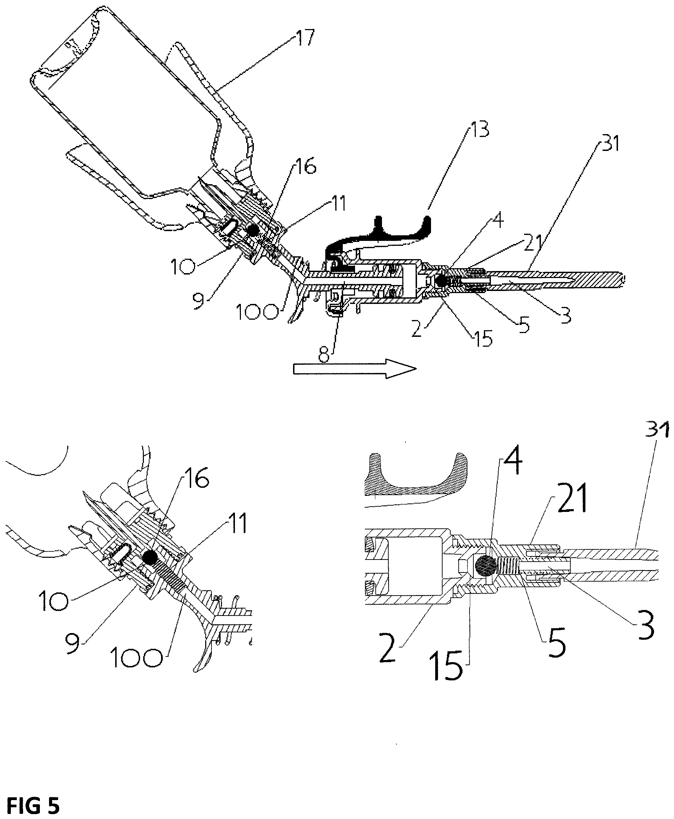

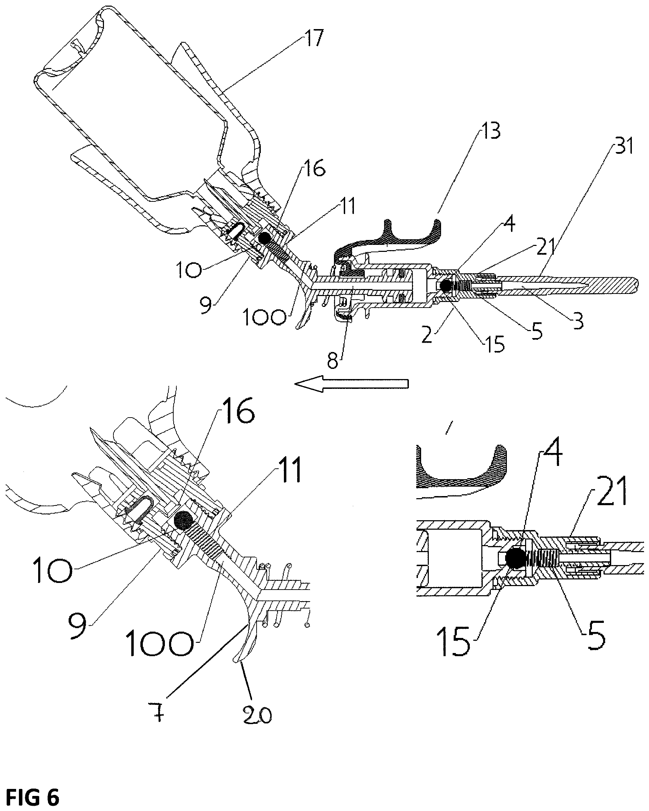

L'organe 4 d'obturation de la sortie 3 d'évacuation de fluide est ouvert, et l'organe 10 d'obturation de la zone 9 tubulaire est fermé quand le piston est déplacé dans le sens d'une rentrée dans le corps de seringue en vue d'une injection, comme l'illustre la figure 5, alors que l'organe 4 d'obturation de la sortie 3 d'évacuation de fluide est fermé et l'organe 10 d'obturation de la zone 9 tubulaire est ouvert quand le piston est déplacé dans le sens d'une sortie du corps de seringue en vue d'un rechargement en fluide issu du réservoir de la chambre du corps de seringue, comme l'illustre la figure 6.

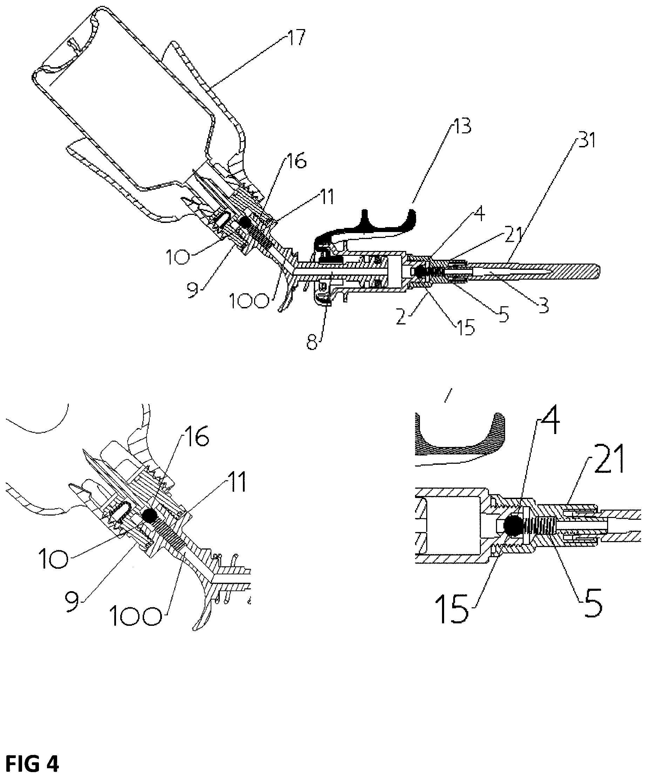

En l'absence de déplacement du piston, les deux organes d'obturation sont en position fermée, comme l'illustre la figure 4.

De manière caractéristique à l'invention, le piston 6 est équipé de moyens 12 de rappel en position partiellement sortie du corps 2 de seringue. Ces moyens 12 de rappel sont ici formés par un simple ressort hélicoïdal disposé entre tête de piston et fond du corps de seringue.

Le corps 2 de seringue est quant à lui bordé longitudinalement d'une oreille 13 formée d'au moins deux ailes 131 radiales externes audit corps, disposées alignées suivant un axe parallèle à l'axe longitudinal du corps de seringue et décalées axialement le long dudit corps. Ces ailes 131 délimitent entre elles une zone 132 d'appui d'au moins un doigt 32 apte à s'enrouler au moins partiellement autour dudit corps 2 de seringue.

La zone 132 d'appui d'au moins un doigt 32 encadrée par les ailes 131 présente une surface d'appui sensiblement parallèle à l'axe longitudinal du corps 2 de seringue. Cette zone 132 forme un appui pour la partie palmaire dudit doigt.

Dans les exemples représentés, l'oreille 13 est disposée sur une pièce 14 rapportée sur le corps 2 de seringue et fixée au corps 2 de seringue, de préférence par vissage. En l'occurrence, cette pièce 14 rapportée se présente sous forme d'une bague taraudée vissable sur l'extrémité proximale du corps de seringue, cette bague étant munie d'une patte longitudinale formant ladite oreille.

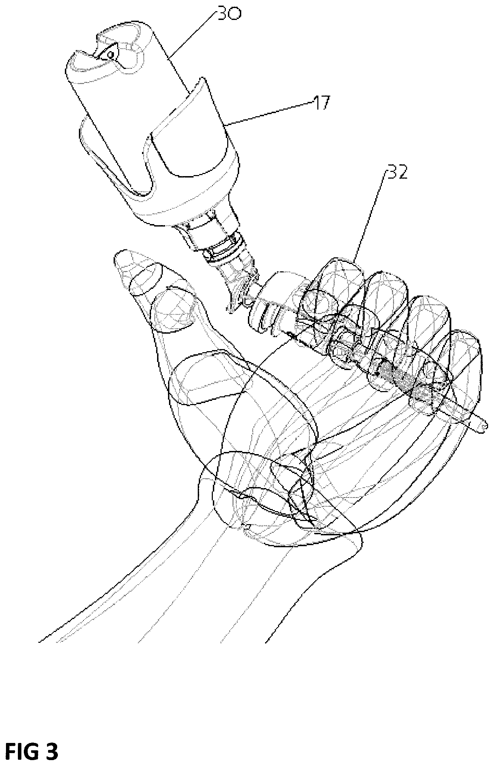

À l'état fixé au corps 2 de seringue, l'oreille est disposée écartée dudit corps 2 de seringue. La main de l'opérateur est ainsi positionnée avec les doigts de l'opérateur qui s'enroulent autour du corps 2 de seringue, comme l'illustre la figure 3, où au moins l'un des doigts est bloqué transversalement entre les deux ailes de l'oreille 13. La main s'étend ici au-delà du corps 2 de seringue et vient également recouvrir une partie de la canule pour permettre, à l'état introduit de la canule dans les naseaux d'un animal, l'appui de la tranche de la main sur le museau de l'animal.

Dans l'exemple représenté, la zone 9 tubulaire et le piston 6 forment entre eux un angle obtus. Cet angle peut être variable suivant les applications. La zone 9 tubulaire se termine, côté raccordement au réservoir, en formant un tube 18 plongeur à extrémité libre chanfreinée sur lequel le réservoir 30 est apte à s'embrocher.

Cette zone 9 tubulaire est encore munie, côté raccordement au réservoir 30, au voisinage de son extrémité libre, d'une douille 17 dite porte-réservoir à l'intérieur de laquelle un réservoir 30 est apte à s'insérer par emmanchement. Cette douille 17 porte-réservoir est ici vissée sur l'extrémité libre de la zone 9 tubulaire.

Enfin, la zone 9 tubulaire est munie, entre son extrémité libre disposée côté raccordement au réservoir et son organe 10 d'obturation, d'au moins un évent 19.

Le poussoir 7 comprend quant à lui, sur une partie de son pourtour, un bras 20 radial externe apte à former une zone d'appui du pouce d'un opérateur. Le bras 20 et l'oreille 13 du corps de seringue sont disposés en regard l'un de l'autre, de manière décalée axialement, suivant l'axe longitudinal du corps 2 de seringue.

En position d'injection, le corps 2 de seringue est disposé sensiblement horizontalement avec l'oreille 13 surmontant le corps 2, l'angle obtus formé entre la zone 9 tubulaire et le piston 6 ouvert vers le ciel et le bras 20 radial du poussoir 7 s'étendant quant à lui en direction du sol.

Lors d'un déplacement du piston dans le sens d'une rentrée dans le corps de seringue, au moins une partie du contenu de la chambre du corps de seringue est expulsée via la sortie 3 d'évacuation de fluide et la canule dans les naseaux de l'animal.

Une fois l'injection opérée, le piston revient automatiquement en position partiellement sortie, grâce à l'action de ses moyens de rappel. Au cours de ce déplacement, une dose de fluide est transférée du réservoir dans la chambre du corps de seringue, via la zone tubulaire et la cavité longitudinale du piston.

Une nouvelle injection par rentrée du piston peut être opérée.

Pendant l'opération d'injection, la main de l'opérateur peut être, par sa tranche, maintenue en contact permanent avec la tête de l'animal.

## Revendications

1. Dispositif (1) d'injection de fluide aux animaux notamment par voie intra-nasale comprenant un corps (2) de seringue tubulaire dont l'extrémité (21) distale est munie d'une sortie (3) obturable d'évacuation de fluide, un piston (6) creux équipé d'un poussoir (7) et monté à coulissement axial dans ledit corps (2) de seringue, ledit piston (6) creux se prolongeant au-delà du poussoir (7) par une zone (9) tubulaire obturable raccordable à un réservoir (30) d'alimentation en fluide à injecter, l'organe (10) d'obturation de la zone (9) tubulaire et l'organe (4) d'obturation de la sortie (3) d'évacuation de fluide, équipés chacun de moyens (11, 5) de rappel en position fermée, étant respectivement configurés pour, à l'état partiellement introduit du piston (6) dans le corps (2) de seringue, passer de la position fermée à la position ouverte l'un (10), sous l'effet du déplacement du piston (6) dans le sens d'une sortie du corps (2) de seringue, l'autre (4), sous l'effet du déplacement du piston (6) dans le sens d'une entrée dans le corps (2) de seringue, le piston (6) étant équipé de moyens (12) de rappel en position partiellement sortie du corps (2) de seringue et **caractérisé en ce que** le corps (2) de seringue est bordé longitudinalement d'une oreille (13) formée d'au moins deux ailes (131) ou nervures radiales externes audit corps (2) disposées de manière écartée l'une de l'autre le long d'un axe sensiblement parallèle à l'axe longitudinal du corps (2) de seringue, l'intervalle entre lesdites ailes (131) ou nervures délimitant une zone (132) d'appui d'au moins un doigt (32) apte à s'enrouler au moins partiellement autour dudit corps (2) de seringue.

2. Dispositif (1) selon la revendication 1,
**caractérisé en ce que** la zone (132) d'appui d'au moins un doigt (32) encadrée par les ailes (131) présente une surface d'appui sensiblement parallèle à l'axe longitudinal du corps (2) de seringue.

3. Dispositif (1) selon l'une des revendications précédentes,
**caractérisé en ce que** l'oreille (13) est disposée sur une pièce (14) rapportée sur le corps (2) de seringue et fixée au corps (2) de seringue de préférence par vissage.

4. Dispositif (1) selon l'une des revendications précédentes,
**caractérisé en ce que** la zone (9) tubulaire et le piston (6) forment entre eux un angle obtus.

5. Dispositif (1) selon la revendication 4,
**caractérisé en ce qu'**en position d'injection, ledit corps (2) de seringue est disposé sensiblement horizontalement avec l'oreille (13) surmontant ledit corps (2) et l'angle obtus formé entre la zone (9) tubulaire et le piston (6) ouvert vers le ciel.

6. Dispositif (1) selon l'une des revendications précédentes,
**caractérisé en ce que** la zone (9) tubulaire se termine, côté raccordement à un réservoir (30), en formant un tube (18) plongeur à extrémité libre chanfreinée ou en forme d'olive sur lequel le réservoir (30) à raccorder est apte à se fixer.

7. Dispositif (1) selon l'une des revendications précédentes,
**caractérisé en ce que** la zone (9) tubulaire est munie, côté raccordement à un réservoir (30), au voisinage de son extrémité libre, d'une douille (17) dite porte-réservoir à l'intérieur de laquelle le réservoir (30) à raccorder est apte à s'insérer.

8. Dispositif (1) selon l'une des revendications précédentes,
**caractérisé en ce que** la zone (9) tubulaire est munie, entre son extrémité libre disposée côté raccordement à un réservoir (30) et son organe (10) d'obturation, d'au moins un évent (19).

9. Dispositif (1) selon l'une des revendications précédentes,
**caractérisé en ce que** l'organe (10) d'obturation de la zone (9) tubulaire et l'organe (4) d'obturation de la sortie (3) d'évacuation de fluide du corps (2) se seringue comprennent chacun une bille, et **en ce que** les cavités formées l'une, par la sortie (3) d'évacuation de fluide, l'autre, par la zone (9) tubulaire comportent chacune un siège (15, 16) de réception dudit organe (4, 10) d'obturation associé.

10. Dispositif (1) selon l'une des revendications précédentes,
**caractérisé en ce que** le poussoir (7) comprend, sur une partie de son pourtour, un bras (20) radial externe apte à former une zone d'appui du pouce d'un opérateur, ledit bras (20) et l'oreille (13) du corps (2) de seringue étant disposés en regard l'un de l'autre de manière décalée axialement suivant l'axe longitudinal du corps (2) de seringue.

## Patentansprüche

1. Vorrichtung (1) zum Einspritzen von Fluid bei Tieren, vor allem auf intranasalem Weg, umfassend einen rohrförmigen Spritzenkörper (2), dessen distales Ende (21) mit einem verschließbaren Fluidabgabeausgang (3) ausgestattet ist, einen hohlen Kolben (6), der mit einem Drücker (7) ausgestattet ist und der axial gleitend in dem Spritzenkörper (2) montiert ist, wobei sich der hohle Kolben (6) mittels einer verschließbaren, an ein Versorgungsreservoir (30) mit einzuspritzendem Fluid gekoppelten rohrförmigen Zone (9) jenseits des Drückers (7) verlängert, wobei das Verschlussorgan (10) der rohrförmigen Zone (9) und das Verschlussorgan (4) des Fluidabgabeausgangs (3), die jeweils mit Rückstellmitteln (11, 5) in geschlossene Position ausgestattet sind, jeweils konfiguriert sind, um im teilweise eingeführten Zustand des Kolbens (6) in den Spritzenkörper (2) von der geschlossenen Position in die geöffnete Position, das eine (10), unter der Wirkung der Verlagerung des Kolbens (6) in die Richtung eines Ausgangs des Spritzenkörpers (2), das andere (4), unter der Wirkung der Verlagerung des Kolbens (6) in die Richtung eines Eingangs in den Spritzenkörper (2), zu wechseln,
wobei der Kolben (6) mit Rückstellmitteln (12) in teilweise ausgefahrene Position des Spritzenkörpers (2) ausgestattet ist und **dadurch gekennzeichnet ist, dass** der Spritzenköper (2) längs von einen Ohr (13) gesäumt ist, das von mindestens zwei radialen Flügeln (131) oder Rippen außerhalb des Körpers (2) gebildet ist, die voneinander beabstandet entlang einer Achse, die etwa parallel zur Längsachse des Spritzenkörpers (2) ist, angeordnet sind, wobei das Intervall zwischen den Flügeln (131) oder Rippen eine Stützzone (132) mindestens eines Fingers (32) begrenzt, der imstande ist, sich mindestens teilweise um den Spritzenkörper (2) zu legen.

2. Vorrichtung (1) nach Anspruch 1,
**dadurch gekennzeichnet, dass** die Stützzone (132) mindestens eines Fingers (32), die von den Flügeln (131) umrahmt ist, eine Stützfläche aufweist, die etwa parallel zur Längsachse des Spritzenkörpers (2) ist.

3. Vorrichtung (1) nach einem der vorangehenden Ansprüche,
**dadurch gekennzeichnet, dass** das Ohr (13) auf einem auf dem Spritzenkörper (2) angebrachten und am Spritzenkörper (2) vorzugsweise durch Schrauben befestigten Teil (14) angeordnet ist.

4. Vorrichtung (1) nach einem der vorangehenden Ansprüche,
**dadurch gekennzeichnet, dass** die rohrförmige Zone (9) und der Kolben (6) zwischen sich einen stumpfen Winkel bilden.

5. Vorrichtung (1) nach Anspruch 4,
**dadurch gekennzeichnet, dass** der Spritzenkörper (2) in Einspritzposition etwa horizontal mit dem Ohr (13) über dem Körper (2) angeordnet und der stumpfen Winkel zwischen der rohrförmigen Zone (9) und dem Kolben (6) in Richtung Himmel geöffnet gebildet ist.

6. Vorrichtung (1) nach einem der vorangehenden Ansprüche,
**dadurch gekennzeichnet, dass** die rohrförmige Zone (9) an der Anschlussseite an ein Reservoir (30) endet, indem sie ein Tauchrohr (18) mit abgeschrägtem freien Ende oder in Olivenform bildet, an welchem das anzuschließende Reservoir (30) imstande ist, sich zu fixieren.

7. Vorrichtung (1) nach einem der vorangehenden Ansprüche,
**dadurch gekennzeichnet, dass** die rohrförmige Zone (9) an der Anschlussseite an ein Reservoir (30) in der Nähe ihres freien Endes mit einer Reservoirträgerhülse (17) ausgestattet ist, in deren Innern das anzuschließende Reservoir (30) imstande ist, sich einzufügen.

8. Vorrichtung (1) nach einem der vorangehenden Ansprüche,
**dadurch gekennzeichnet, dass** die rohrförmige Zone (9) zwischen ihrem freien Ende, das an der Anschlussseite an ein Reservoir (30) angeordnet ist, und ihrem Verschlussorgan (10) mit mindestens einem Luftloch (19) ausgestattet ist.

9. Vorrichtung (1) nach einem der vorangehenden Ansprüche,
**dadurch gekennzeichnet, dass** das Verschlussorgane (10) der rohrförmigen Zone (9) und das Verschlussorgan (4) des Fluidabgabeausgangs (3) des Spritzenkörpers (2) jeweils eine Kugel umfassen, und dass die Hohlräume, die, der eine, vom Fluidabgabeausgang (3), der andere, von der rohrförmigen Zone (9), gebildet sind, jeweils einen Empfangssitz (15, 16) des zugeordneten Verschlussorgans (4, 10) aufweisen.

10. Vorrichtung (1) nach einem der vorangehenden Ansprüche,
**dadurch gekennzeichnet, dass** der Drücker (7) auf einem Abschnitt seines Umfangs einen externen radialen Arm (20) umfasst, der imstande ist, eine Stützzone eines Daumens eines Bedieners zu bilden, wobei der Arm (20) und das Ohr (13) des Spritzenkörpers (2) einander gegenüber gemäß der Längsachse des Spritzenkörpers (2) axial versetzt angeordnet sind.

## Claims

1. A device (1) for injecting fluid in animals, in particular by intranasal administration, comprising a tubular syringe body (2) whereof the distal end (21) is provided with a fluid discharge outlet (3) that is able to be closed off, a hollow plunger (6) equipped with a push-piece (7) is mounted axially sliding in said syringe body (2), said hollow plunger (6) extending beyond the push-piece (7) by a closable tubular zone (9) connectable to a reservoir (30) supplying fluid to be injected, the member (10) closing off the tubular zone (9) and the member (4) closing off the fluid discharge outlet (3), each equipped with means (11, 5) for returning to the closed position, respectively being configured so as, in the state with the plunger (6) partially inserted in the syringe body (2), to go from the closed position to the open position, one (10) under the effect of the movement of the plunger (6) in the direction of an exit from the syringe body (2), the other (4) under the effect of a movement of the plunger (6) in the direction of an entry into the syringe body (2), the plunger (6) being equipped with means (12) returning to the position partially outside the syringe body (2), and
**characterized in that** the syringe body (2) is longitudinally bordered by a lug (13) formed by at least two flanges (131) or radial ribs outside said body (2) arranged so as to be separated from one another along an axis substantially parallel to the longitudinal axis of the syringe body (2), the interval between said flanges (131) or ribs defining a bearing zone (132) for at least one finger (32) able to wind at least partially around said syringe body (2).

2. The device (1) according to claim 1,
**characterized in that** the bearing zone (132) for at least one finger (32) framed by the flanges (131) has a bearing surface substantially parallel to the longitudinal axis of the syringe body (2).

3. The device (1) according to one of the preceding claims,
**characterized in that** the lug (13) is arranged on a part (14) attached on the syringe body (2) and fixed to the syringe body (2), preferably by screwing.

4. The device (1) according to one of the preceding claims,
**characterized in that** the tubular zone (9) and the plunger (6) together form an obtuse angle.

5. The device (1) according to claim 4,
**characterized in that** in the injection position, said syringe body (2) is arranged substantially horizontally with the lug (13) topping said body (2) and the obtuse angle formed between the tubular zone (9) and the plunger (6) opening toward the sky.

6. The device (1) according to one of the preceding claims,
**characterized in that** the tubular zone (9) ends, on the side connected to a reservoir (30), by forming a plunger tube (18) with a beveled or diamond-shaped free end on which the reservoir (30) to be connected can be fixed.

7. The device (1) according to one of the preceding claims,
**characterized in that** the tubular zone (9) is provided, on the side connected to a reservoir (30), near its free end, with a so-called reservoir support bush (17) inside which the reservoir (30) to be connected can be inserted.

8. The device (1) according to one of the preceding claims,
**characterized in that** the tubular zone (9) is provided, between its free end arranged on the side connecting to a reservoir (30) and its closing off member (10), with at least one vent (19).

9. The device (1) according to the preceding claims,
**characterized in that** the member (10) closing off the tubular zone (9) and the member (4) closing off the fluid discharge outlet (3) of the syringe body (2) each comprise a bead, and **in that** the cavities, one of which is formed by the fluid discharge outlet (3) and the other of which is formed by the tubular zone (9), each include a seat (15, 16) for receiving said associated closing off member (4, 10).

10. The device (1) according to one of the preceding claims,
**characterized in that** the push-piece (7) comprises, on part of its perimeter, an outer radial arm (20) able to form a bearing zone for the thumb of an operator, said arm (20) and the lug (13) of the syringe body (2) being arranged across from one another axially offset along the longitudinal axis of the syringe body (2).
